# EUROPEAN PATENT APPLICATION

(11) **EP 3 153 138 A1**
(43) Date of publication of application: **12.04.2017**
(21) Application number: 15806917.9
(22) Date of filing: 08.06.2015
(51) Int. Cl.: A61F 7/10

(54) **BODY SURFACE TEMPERATURE ADJUSTMENT DEVICE**

(30) Priority: 09.06.2014 JP 2014118256
(71) Applicant: Endo, Yasuhiro, Ashiya-shi, Hyogo 659-0085 (JP)
(72) Inventor: Endo, Yasuhiro, Ashiya-shi, Hyogo 659-0085 (JP)
(74) Representative: von Bülow & Tamada
(86) International application number: PCT/JP2015/066485
(87) International publication number: WO 2015/190436

(57) **Abstract**

A body surface temperature adjustment apparatus efficiently adjusts a surface temperature of a treatment target site of a patient. The apparatus includes a first tank (31) that stores water (3), a water temperature adjustment unit (34a) that adjusts a temperature of the water (3) to a predetermined temperature, a cooling apparatus (41) that surrounds a scalp (2a), a first pump (32) that circulates the water (3), a controller (62) that controls the water temperature adjustment unit (34a) and the first pump (32), and at least one water outlet (42) for releasing the water (3) circulated by the first pump (32) toward the scalp (2a) surrounded by the cooling apparatus body (41), and a collection unit (50) that collects the water (3) released within a scalp surrounding space (41c) into the first tank (31).

## Description

### FIELD

The present invention relates to a body surface temperature adjustment apparatus that cools a body part such as the scalp to an intended temperature.

### BACKGROUND

Patients who have received chemotherapy treatment such as the administration of anticancer drugs may experience side effects of anticancer drugs such as hair loss and nail discoloration, which have been considered as one of major challenges in anticancer therapy.

For example, such hair loss may occur when an anticancer drug impairs the activity of hair follicle cells forming hair roots.

In detail, actively dividing cancer cells are sensitive to anticancer drugs, and their cell dividing activities are impaired, hence inhibition of the growth of the cancer.

Cells that form some body parts including hair roots and nails also divide actively. Thus, anticancer drugs can also impair such normal cells forming body parts including hair roots and nails, and may cause side effects such as hair loss and nail discoloration.

These side effects are expected to be prevented by lowering the activity level of such cells forming body parts including hair roots and nails, or by constricting capillary vessels that supply blood in order to reduce the exposure level of anticancer drugs to such cells forming hair roots and nails.

More specifically, effective methods for reducing side effects can be to cool body parts including the scalp and nails in order to reduce the cell dividing activity of the cells forming such parts including hair roots and nails, or to constrict the capillary vessels across the scalp to reduce the amount of anticancer drugs reaching the cells forming such parts including hair roots and nails. For example, Patent Literature 1 describes a head cooling unit for cooling the scalp.

The head cooling unit described in Patent Literature 1 includes a head cooling cap with silicon tubes incorporated, which are supposedly closely fitted to the scalp to form the closed circuit over the scalp. A refrigerated liquid circulates through the silicon tubes across the scalp to cool the scalp of a patient wearing such a scalp cooling cap.

However, the scalp cooling cap can allow hairs or an air layer to be present at the space between the cooling cap and the scalp. A gas including the air has a much lower thermal conductance value than that of a solid or a liquid. Although the head cooling unit described in Patent Literature 1 can cool the scalp, the cooling efficiency of this head cooling unit may not high and have difficulties in maintaining and controlling the scalp at the target temperature.

### CITATION LIST

### PATENT LITERATURE

Patent Literature 1: Japanese Unexamined Patent Application Publication No. 2014-23604

### SUMMARY

### TECHNICAL PROBLEM

It is an object of the present invention to provide a body surface temperature adjustment apparatus that efficiently adjusts the temperature of the body surfaces of treatment target sites of a patient, such as the scalp and nails.

### SOLUTION TO PROBLEM

One aspect of the present invention provides a patient adjustment apparatus for adjusting a surface temperature of a target site of a patient to a predetermined temperature. The apparatus includes a storage tank that stores a fluid, a temperature adjustment unit that adjusts a temperature of the fluid to the desired, predetermined temperature, an enclosure that surrounds the head of a patient (i.e., target site), a circulation unit that circulates the fluid, a controller unit that controls the temperature adjustment unit and the circulation circuit, at least one releasing part that releases the fluid circulated by the circulation unit toward a patient site surrounded by the enclosure unit, and a collection unit that collects the fluid released inside the space surrounded by the enclosure and transfers fluid into the storage tank.

The treatment target site includes body parts of a patient such as hair roots and nails with active cell division capability, and specifically includes body parts such as the scalp, finger ends, toes, and areas around eyelids.

The fluid, which is flowable, includes a liquid, a gas, and a gel, and further includes a mist of fine droplets of a liquid dispersed in the air, a particulate powder of a solid such as ice, and their mixture.

The temperature adjustment unit may be a temperature adjustment unit including a cooling or heating unit for cooling or heating a fluid to a predetermined temperature in accordance with intended use of the body surface temperature adjustment apparatus (e.g., anticancer drug treatment, low-temperature therapy, or treatment for soothing muscle or alleviating stiffness), and may be a temperature adjustment unit with an additional mechanism for detecting the temperature of a fluid, such as a temperature sensor, for adjusting the temperature of the fluid to a predetermined temperature.

The circulation unit and the collection unit include a pump and a motor for maintaining circulation or collection of the fluid, and further a hose for guiding the fluid to the releasing parts or to the storage tank.

The apparatus according to the aspect of the present invention efficiently adjusts the surface temperature of a treatment target site.

In detail, the fluid released toward the body surface of the treatment target site of the patient comes in a direct contact with the body surface of the treatment target site. Thus, the surface temperature can be efficiently adjusted to a predetermined temperature without allowing air to be present between the body surface of the treatment target site and the fluid.

In this manner, for example, the scalp of a patient who undergoes chemotherapy treatment can be cooled and is maintained at a predetermined temperature to avoid anti-cancer drug-induced injuries to cells forming the hair roots, and it is expected that the side effects caused by the administration of the anticancer drugs such as hair loss as described above will be reduced.

Further, the surface temperature adjustment apparatus includes the collection unit for collecting the fluid. This structure reuses the fluid without wasting it, and efficiently preserves thermal energy of the collected fluid to efficiently adjust the surface temperature.

In detail, without collecting the fluid released from the releasing parts, an amount of fluid equal to the amount of the released fluid is to be added to the storage tank as appropriate. The added fluid would change the temperature of the fluid in the storage tank. Thermal energy is then needed for adjusting the temperature of the fluid in the storage tank to a predetermined temperature.

The body surface temperature adjustment apparatus including the collection unit according to the aspect of the present invention collects the released fluid into the storage tank without wasting it but reuses the fluid. In other words, the fluid is circulated to minimize the amount of thermal energy needed for maintaining and adjusting the temperature of the fluid to a predetermined temperature. The fluid can be reused, and thus fresh fluid is not required.

Thus, the body surface temperature adjustment apparatus efficiently adjusts the body surface temperature by releasing the fluid directly onto the body surface of the treatment target site, and collects and reuses the released fluid without wasting the fluid, and uses the thermal energy to efficiently adjust the temperature of the fluid to a predetermined temperature.

In the body surface temperature adjustment apparatus according to the aspect of the present invention, the fluid comes in a direct contact with the body surface of the treatment target site without using silicon tubes placed between the scalp and the liquid, unlike in the head cooling unit described in Patent Literature 1. The body surface temperature adjustment apparatus according to the aspect of the present invention allows more effective temperature adjustment than the scalp cooling unit described in Patent Literature 1.

Thus, the body surface temperature adjustment apparatus according to the aspect of the present invention allows efficient treatment through the administration of anticancer drugs by, for example, shortening the time needed to cool the scalp to a predetermined temperature before the administration of anticancer drugs, and thus reduces burdens on a patient.

In the apparatus according to another aspect of the invention, the at least one releasing part includes a plurality of releasing parts, and the plurality of releasing parts are arranged to release the fluid toward the treatment target site in multiple directions.

In the apparatus according to the aspect of the present invention, the fluid can be released onto a wider area of the treatment target site, and thus more efficiently adjusts the surface temperature of the treatment target site.

The amount and the temperature of the fluid to be released from the releasing parts are adjustable in accordance with the part with which the fluid released from the releasing parts comes in direct contact to adjust the temperature. This prevents failure such as excessive cooling, and adjusts the surface temperature of the treatment target site in a more reliable and efficient manner.

In the apparatus according to another aspect of the present invention, the at least one releasing part is movable relative to the treatment target site.

In the apparatus according to the aspect of the present invention, the small number of releasing parts can release the fluid onto a wider area of the treatment target site by moving. This apparatus thus more efficiently adjusts the surface temperature of the treatment target site.

The apparatus according to another aspect of the invention further includes a fluid-releasing amount controlling unit that sets a releasing amount of the fluid. The controller is driven in accordance with the releasing amount set by the fluid-releasing amount control unit.

The apparatus according to the aspect of the present invention performs efficient temperature adjustment that is appropriate for a patient or for the treatment target site.

In detail, the difference between the predetermined temperature and the surface temperature varies depending on the patient or the treatment target site. When, for example, the temperature difference is large, a large amount of fluid may be released. Controlling the releasing amount appropriately for a patient or for the treatment target site thus enables efficient temperature adjustment.

For example, when the treatment target site is the scalp, the scalp temperature of the top part of the head may be more difficult to adjust than the scalp temperature of the side parts of the head, which are easy to cool. In this case, a large amount of fluid can be released onto the top part of the head by adjusting the amount of fluid released from the releasing parts. This easily adjusts the scalp temperature of the top part of the head, in which the temperature of the scalp is difficult to adjust.

As described above, the amount of fluid to be released from the releasing parts is adjustable. The amount of fluid to be released is adjusted in accordance with a patient or the treatment target site to efficiently adjust the surface temperature.

The apparatus according to another aspect of the invention further includes a fluid releasing duration controlling unit that can adjust a fluid releasing time. The controller is driven in accordance with the fluid releasing time set by the fluid releasing controlling unit.

The apparatus according to the aspect of the present invention can set the duration time for adjusting the surface temperature appropriately in accordance with a patient or the treatment target site.

In detail, although the appropriate duration for surface temperature adjustment by the surface temperature adjustment apparatus varies depending on a patient or a treatment target site, the fluid releasing controlling unit sets the fluid circulation time of the fluid for adjusting the surface temperature in accordance with a patient or a treatment target site. Thus, the surface temperature can be adjusted for the optimum duration for a patient or a treatment target site, which minimizes burdens on the patient.

Further, the amount of fluid released or the fluid temperature can be set in accordance with the set duration time. In this case, for example, the temperature can be adjusted in accordance with the blood concentration of an anticancer drug after the drug administration.

In the apparatus according to another aspect of the invention, the temperature adjustment unit is the first temperature adjustment unit, and the collection unit includes a collection tank that collects the fluid, and a second temperature adjustment unit that adjusts a temperature of the fluid in the collection tank.

The apparatus according to the aspect of the present invention allows the fluid to be collected into the storage tank through the collection tank and the second temperature adjustment unit. The temperature of the fluid in the storage tank can thus be constantly maintained at a predetermined temperature.

In detail, when the released fluid is directly collected into the storage tank, the fluid temperature may change while the fluid is circulating through the body surface temperature adjustment apparatus depending on the amount of the released fluid or other conditions of use. In that case, the fluid temperature of the fluid in the storage tank can significantly deviate from a predetermined temperature.

In the structure with the collection unit including the collection tank and the second temperature adjustment unit, the fluid in the collection tank can be adjusted to around a predetermined temperature. This prevents the temperature of the fluid in the storage tank to significantly deviate from the predetermined temperature by the fluid fed to the storage tank. In other words, the fluid in the storage tank can be maintained at a constant temperature.

Thus, the fluid circulating from the storage tank toward the releasing parts by the circulation unit can constantly be at a predetermined temperature to efficiently adjust the surface temperature of the treatment target site to a predetermined temperature.

In the apparatus according to another aspect of the present invention, at least one of the target site enclosure or the collection unit includes a filter that filters the fluid.

The apparatus according to the aspect of the present invention removes impurities contained in the fluid to be collected, and maintains the insides of the storage tank to be clean, and repeatedly releases the clean fluid onto the treatment target site. The apparatus can also prevent the pumps included in the circulation unit from being clogged with long hairs and being broken, or from lowering the circulation efficiency.

In the structure including the collection tank with the filter, the collection tank including the second temperature adjustment unit serves as a sub tank for the storage tank when the circulation efficiency decreases. This prevents failures such as insufficient release of the fluid due to lack of the fluid that has been adjusted to a predetermined temperature.

In the apparatus according to another aspect of the invention, the predetermined temperature is lower than a body temperature of the treatment target site, the fluid is water, and the treatment target site is a scalp.

The apparatus according to the aspect of the present invention uses water, which is inexpensive and has a high thermal conductance, as a medium for cooling the scalp to a predetermined temperature. As described above, the apparatus thus reduces hair loss, which is a side effect of the administration of anticancer drugs, at low cost.

Another aspect of the present invention provides a body surface temperature adjustment apparatus including a treatment bed. The apparatus includes the body surface temperature adjustment apparatus described above, and a treatment bed that allows the patient to lie on horizontally. The body surface temperature adjustment apparatus is arranged to be adjacent to a head of the patient lying horizontally on the treatment bed.

The treatment bed includes a chair or a bed that allows the patient to lie on horizontally in a comfortable posture.

The apparatus according to the aspect of the present invention adjusts the body surface temperature of the treatment target site without placing burdens on the patient during the treatment.

### ADVANTAGEOUS EFFECTS

The adjustment apparatus according to embodiments of the present invention efficiently adjusts the surface temperature of treatment target sites of a patient, such as the scalp and nails.

### BRIEF DESCRIPTION OF THE DRAWINGS

Fig. 1 is a perspective view of a scalp cooling system.
Fig. 2 is a schematic diagram of the scalp cooling system.
Fig. 3 is a schematic perspective view of a scalp cooling apparatus.
Fig. 4 is a plan view of the scalp cooling apparatus in use.
Fig. 5 is a block diagram of the scalp cooling apparatus.
Fig. 6 is a flowchart for the scalp cooling apparatus.
Fig. 7 is a flowchart for the scalp cooling apparatus.

### DETAILED DESCRIPTION

An embodiment of the present invention will now be described with reference to the drawings.

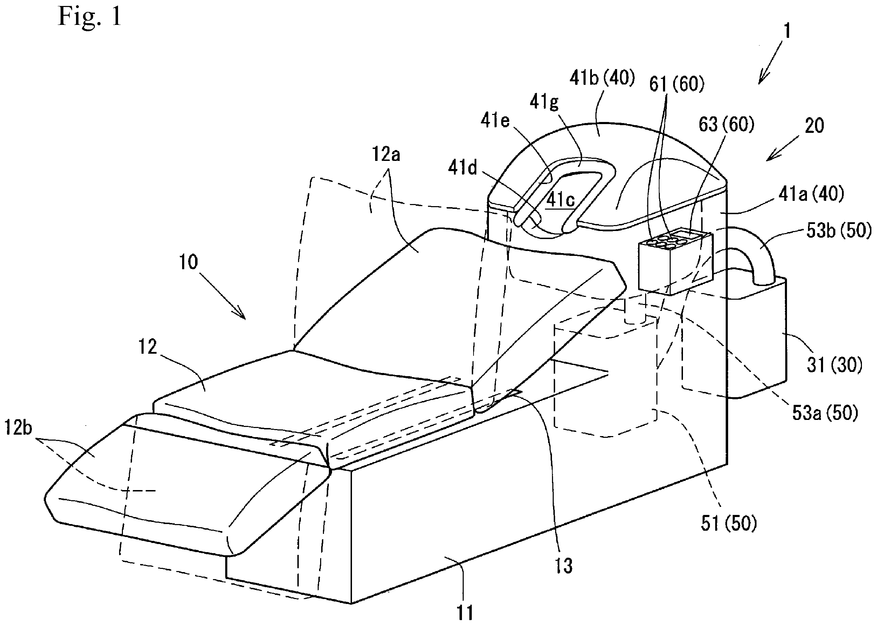

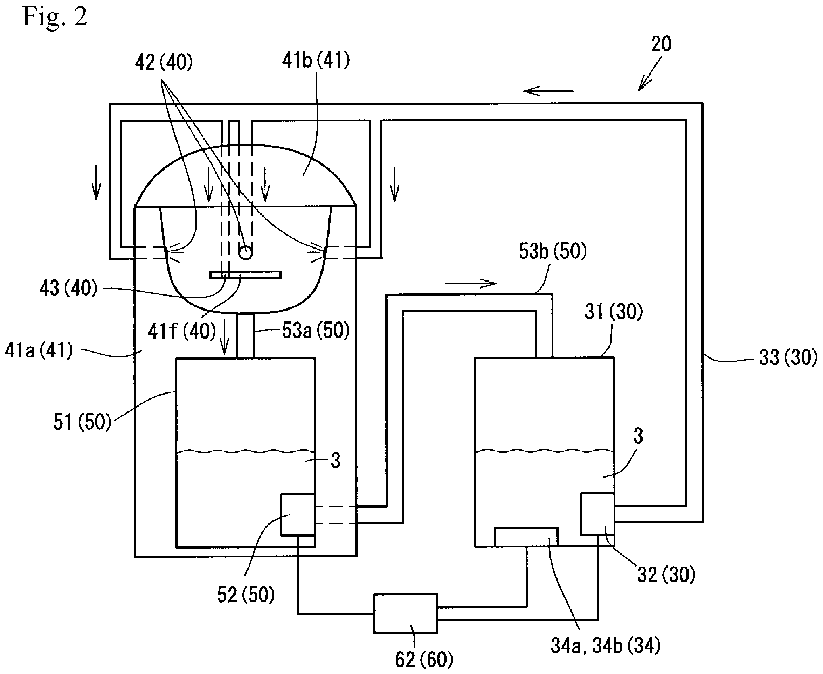

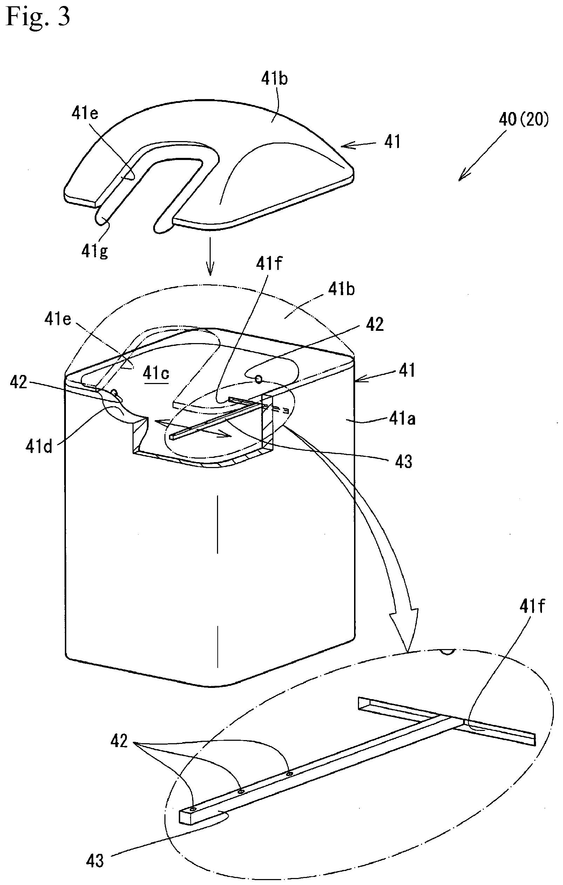

Fig. 1 is a perspective view of a scalp cooling system 1. Fig. 2 is a schematic diagram of the scalp cooling system 1. Fig. 3 is a schematic perspective view of a scalp cooling apparatus 20.

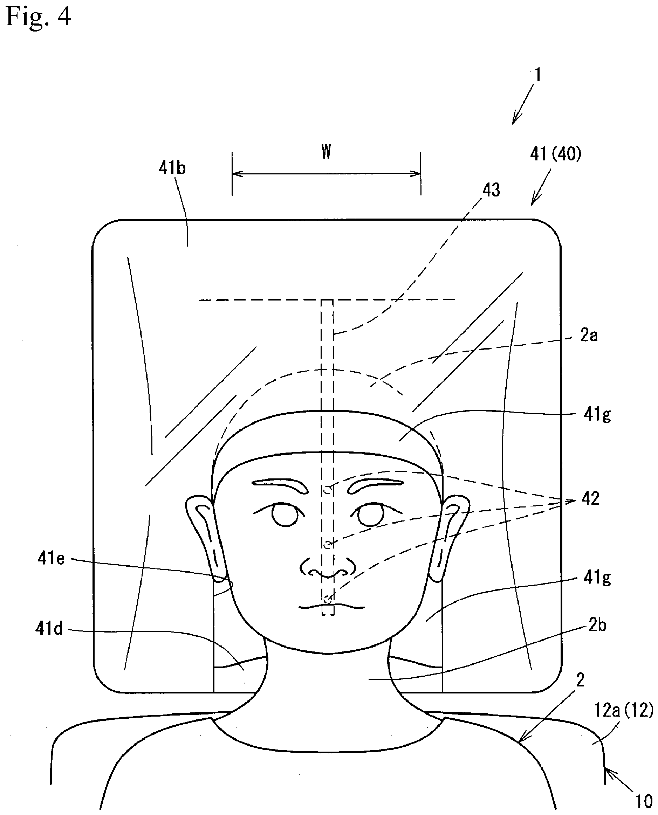

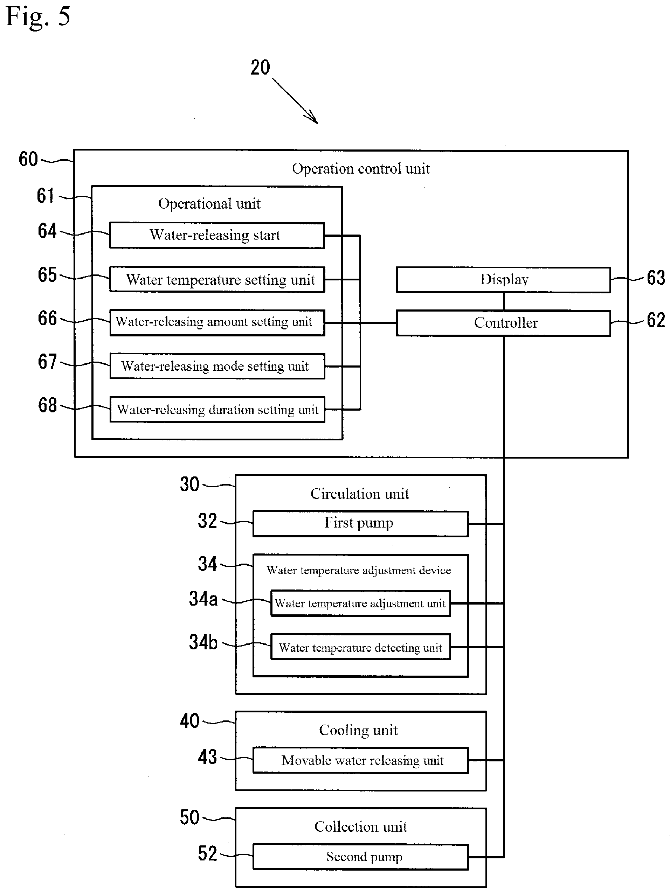

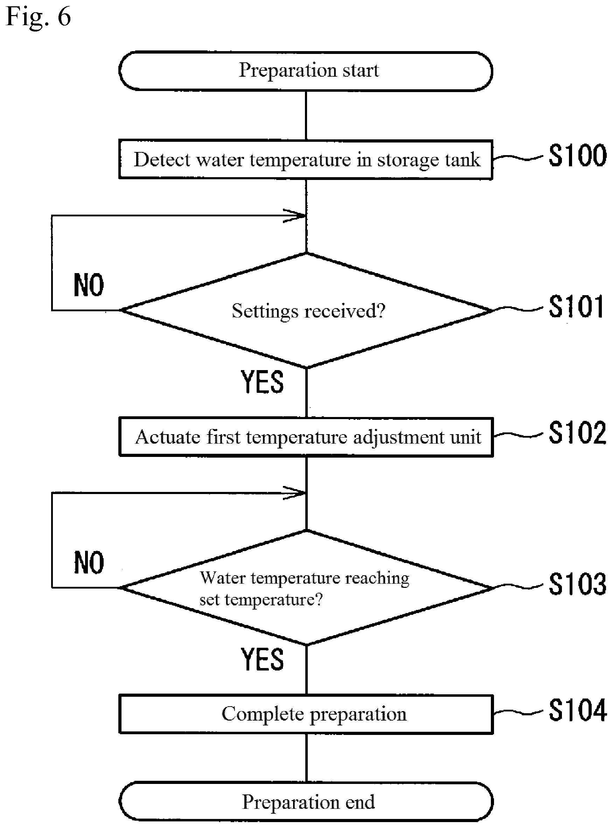

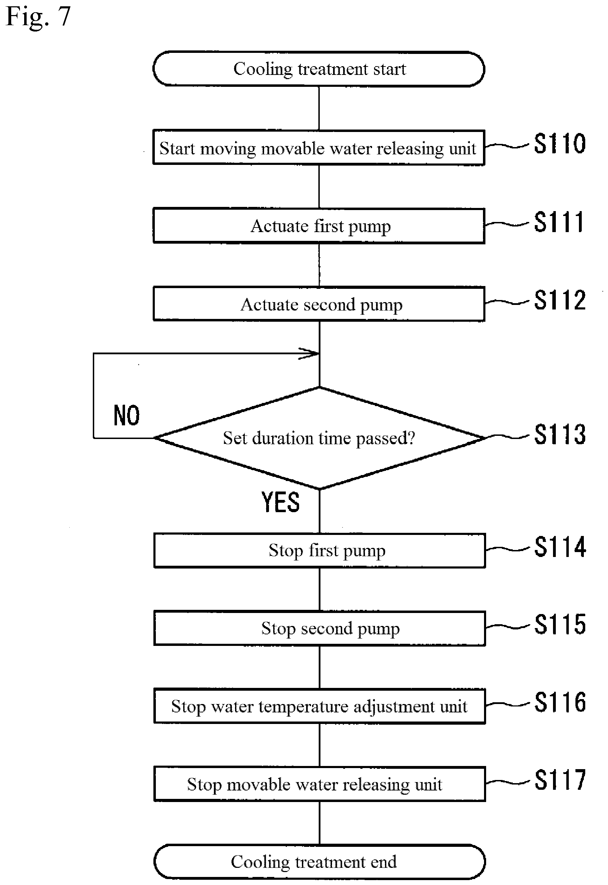

Fig. 4 is a plan view of the scalp cooling apparatus 20 in use. Fig. 5 is a block diagram of the scalp cooling apparatus 20. Fig. 6 is a flowchart showing a preparation process performed by the scalp cooling apparatus 20. Fig. 7 is a flowchart showing a cooling treatment process performed by the scalp cooling apparatus 20.

As shown in Fig. 1, the scalp cooling system 1 according to the present embodiment includes a treatment bed 10, on which a patient 2 is to lie horizontally, and the scalp cooling apparatus 20, which cools the scalp 2a of the patient 2 to a predetermined temperature.

The treatment bed 10 and the scalp cooling apparatus 20 are arranged to allow the patient 2 to lie on the treatment bed 10 with his or her head being placed adjacent to the scalp cooling apparatus 20.

The treatment bed 10 includes a base 11, and a bed body 12 mounted on the base 11.

The base 11 elongates longitudinally, and is substantially rectangular as viewed from above. The base 11 has the scalp cooling apparatus 20 arranged on its one end in the longitudinal direction, and the bed body 12 on the other end. The base 11 includes rails 13, which enable the bed body 12 to move relative to the base 11. In the present embodiment, the base 11 and the scalp cooling apparatus 20 are combined integrally.

The bed body 12 includes a backrest 12a and a footrest 12b, which can be reclined or raised. The bed body 12 can hold the body of the patient 2 horizontally when the backrest 12a is reclined and the footrest 12b is raised.

The bed body 12 is mounted on the rails 13 in a manner movable relative to the base 11. The treatment bed 10 may be a common reclining sofa, or instead a chair or a treatment bed that can hold the patient 2 in a comfortable posture.

The rails 13 are two rails that extend in the longitudinal direction of the base 11 as viewed from above and are parallel to each other in the width direction of the base 11. The rails 13 allow the bed body 12 mounted on them to be movable relative to the base 11 in the longitudinal direction, and allow the bed body 12 to move relative to the scalp cooling apparatus 20, which is arranged on one end of the rails 13.

The scalp cooling apparatus 20 intends to reduce hair loss, which is a side effect caused by the administration of an anticancer drug, by releasing water 3, which has been adjusted to a predetermined temperature, onto the scalp 2a of the patient 2 and cooling the scalp 2a to a predetermined temperature. As shown in Fig. 2, the scalp cooling apparatus 20 includes a circulation unit 30, which circulates the water 3 adjusted to a predetermined temperature, a cooling unit 40, which releases the water 3 onto the scalp 2a, a collection unit 50, which collects the released water 3 into the circulation unit 30, and an operation control unit 60, which adjusts and controls the amount or the temperature of the water 3 to be released by the cooling unit 40.

The circulation unit 30 includes a first tank 31 for storing the water 3, a first pump 32 for circulating the water 3 toward the cooling unit 40, a first pipe 33 for connecting the first tank 31 with the cooling unit 40 to guide the circulating water 3 to the cooling unit 40, and a water temperature adjustment device 34 for adjusting the water 3 in the first tank 31 to a predetermined temperature.

The first tank 31 is substantially rectangular, and has a size to store the amount of water 3 enough to circulate through the scalp cooling apparatus 20.

The first pump 32 includes a motor (not shown), and is arranged in the first tank 31. The first pump 32 controls the motor to allow an intended amount of water 3 to circulate toward the cooling unit 40, and adjusts the circulation amount of water 3.

The first pipe 33 is flexible and has a substantially circular cross-section. The pipe is thick enough to allow an intended amount of water 3 to circulate toward the cooling unit 40. The first pipe 33 connects the first tank 31 and the cooling unit 40, and guides the circulating water 3 to the cooling unit 40. The first pipe 33 may be a metal pipe or a synthetic resin pipe.

The water temperature adjustment device 34, which is arranged in the first tank 31, includes a water temperature adjustment unit 34a for adjusting the temperature of the water 3 stored in the first tank 31 to a predetermined temperature, and a water temperature detecting unit 34b for detecting the water temperature of the water 3 in the first tank 31. The water temperature adjustment device 34 is controlled to maintain the water 3 in the first tank 31 at a predetermined temperature.

The cooling unit 40 includes a cooling apparatus body 41, which surrounds the scalp 2a of the patient 2, and water outlets 42 and a movable water releasing unit 43, which are arranged in the cooling apparatus body 41.

As shown in Fig. 3, the cooling apparatus body 41 includes a base portion 41a, and a cover 41b attachable to the base portion 41a. The assembled cooling apparatus body 41 combining the base portion 41a and the cover 41b defines a scalp surrounding space 41c for surrounding the scalp 2a.

The cooling apparatus body 41 includes a neck fitting portion 41d, which is a cut in the base portion 41a, and an outline cut portion 41e, which is a cut in the cover 41b. In the assembled cooling apparatus body 41 surrounding the scalp 2a, the neck fitting portion 41d can fit on a neck 2b, which is at a basal end of the scalp 2a, and the outline cut portion 41e can match the outline of the head.

As shown in the enlarged view of Fig. 3, the base portion 41a has a groove 41f on one inner surface facing the top part of the head of the patient 2, among the inner surfaces defining the scalp surrounding space 41c. The groove 41f is located lower than the back part of the head, and extends in the width direction of the base 11 as viewed from above.

The cover 41b includes a sealing sheet 41 g, which prevents released water 3 from splashing out of the scalp surrounding space 41c. The sealing sheet 41g is flexible, and closely fits on the outline of the patient 2 when the base portion 41a and the cover 41b are combined as shown in Fig. 4.

As shown in Fig. 3, the cooling apparatus body 41 with the above structure has the water outlets 42 in the scalp surrounding space 41c, which are holes with a small diameter for releasing water 3 guided by the first pump 32. The cooling apparatus body 41 has multiple water outlets 42 in its inner surface.

More specifically, the cooling apparatus body 41 has one water outlet 42 in the surface facing the top part of the head of the patient 2 and in each of the surfaces facing the side parts of the head, among the inner surfaces of the base portion 41a defining the scalp surrounding space 41c, and further has three water outlets in the movable water releasing unit 43 (described below).

As shown in the enlarged view of Fig. 3 and in Fig. 4, the movable water releasing unit 43 is a substantially quadrangular rod that elongates longitudinally. The movable water releasing unit 43 has its basal end attachable to the groove 41f, and is horizontally movable along the groove 41f by a distance W.

The movable water releasing unit 43 has the three water outlets 42 in a front portion of its top surface at predetermined intervals in the longitudinal direction.

The movable water releasing unit 43 is moved horizontally by a drive unit (not shown).

As shown in Fig. 2, the collection unit 50 includes a second tank 51 for collecting the released water 3, a second pump 52 for circulating the water 3 toward the first tank 31, a second pipe 53a connecting the cooling apparatus body 41 and the second tank 51 to collect the water 3 into the second tank 51, and a third pipe 53b connecting the second tank 51 and the first tank 31 to guide the circulating water 3 toward the first tank 31.

The second tank 51 is the same as the first tank 31, and will not be described in detail.

The second pump 52 is the same as the first pump 32, and will not be described in detail. The second pump 52 is arranged in the second tank 51.

The second pipe 53a and the third pipe 53b are the same as the first pipe 33, and will not be described in detail.

As shown in Fig. 1, the operation control unit 60 is arranged on a side surface of the cooling apparatus body 41. As shown in Figs. 2 and 5, the operation control unit 60 includes an operational unit 61, which is used to perform the controlling operations of the scalp cooling apparatus 20, a controller 62, which controls the first pump 32, the water temperature adjustment device 34, the movable water releasing unit 43, and the second pump 52 based on the operations of the operational unit 61, and a display 63, which displays information associated with these components.

The operational unit 61 includes a water-releasing start controlling unit 64, a water temperature controlling unit 65, a water-releasing amount controlling unit 66, a water-releasing mode controlling unit 67, and a water-releasing duration controlling unit 68.

In detail, the water-releasing start controlling unit 64 receives an operation to start releasing the water 3 from the water outlets 42. Based on the received operation, the controller 62 starts releasing the water 3 from the water outlets 42.

The water temperature controlling unit 65 is an operational unit for controlling the temperature of the water 3 in the first tank 31, and receives the controlling of the water temperature for the water 3 in the first tank 31. Based on the received controlling, the controller 62 actuates the water temperature adjustment unit 34a to adjust the temperature of the water 3.

The water-releasing amount controlling unit 66 is an operational unit for controlling the amount of water 3 to be released from the water outlets 42, and receives the controlling for adjusting the amount of water 3 to be released onto intended parts of the scalp 2a. Based on the received controlling, the controller 62 controls the number of revolutions of the motor included in the first pump 32 to control the amount of the circulating water 3 to be released from the water outlets 42.

The water-releasing mode controlling unit 67 receives the controlling for the water-releasing mode, or for example the controlling to release the water 3 intermittently from the water outlets 42 or to increase or decrease the moving speed of the movable water releasing unit 43. Based on the received controlling, the controller 62 controls the number of revolutions of the motor in the first pump 32 to control the amount of circulating water 3 to be released from the water outlets 42, and controls the drive unit included in the movable water releasing unit 43 to control the moving speed of the movable water releasing unit 43.

The water-releasing duration controlling unit 68 receives the controlling for the water-releasing duration time for the water 3 released from the water outlets 42. Based on the received controlling, the controller 62 controls the operating time of the first pump 32 to control the water-releasing duration time for the circulating water 3 to be released from the water outlets 42.

As described above, the controller 62 controls the first pump 32, the water temperature adjustment device 34, the movable water releasing unit 43, and the second pump 52 to implement the operations set by the water-releasing start controlling unit 64, the water temperature controlling unit 65, the water-releasing amount controlling unit 66, the water-releasing mode controlling unit 67, and the water-releasing duration controlling unit 68 included in the operational unit 61.

The display 63 displays the temperature of the water 3 in the first tank 31 detected by the water temperature detecting unit 34b, the water temperature of the water 3 in the first tank 31 set by the water temperature controlling unit 65, the amount of water 3 to be released from the water outlets 42 by the first pump 32, the moving speed of the movable water releasing unit 43, the water-releasing mode for intermittently releasing the water 3, or the water-releasing duration time set by the water-releasing duration controlling unit 68.

In the scalp cooling apparatus 20 with the above structure, as shown in Fig. 2, the controller 62 controls the circulation unit 30, the cooling unit 40, and the collection unit 50 based on the operations set by the operational unit 61 to circulate the water 3 from the circulation unit 30, the cooling unit 40, and to the collection unit 50 in this order.

A method for cooling the scalp using the scalp cooling apparatus 20 with the above structure will now be described.

A preparation process before the use of the scalp cooling apparatus 20 will first be described with reference to Fig. 6.

A predetermined amount of water 3 that can circulate in the scalp cooling apparatus 20 is placed in the first tank 31. The scalp cooling apparatus 20 is then powered on.

The scalp cooling apparatus 20, which is powered on, detects the temperature of the water 3 in the first tank 31 using the water temperature detecting unit 34b (step S100).

The scalp cooling apparatus 20 waits until it receives the controls for the water temperature, the water releasing amount, the water-releasing mode, and the water-releasing duration time using the water temperature controlling unit 65, the water-releasing amount controlling unit 66, the water-releasing mode controlling unit 67, and the water-releasing duration controlling unit 68 in the operational unit 61 (No in step S101). When the scalp cooling apparatus 20 receives these controls (Yes in step S101), the apparatus actuates the water temperature adjustment unit 34a (step S102).

The water temperature adjustment unit 34a heats or cools the water 3 based on the temperature of the water 3 stored in the first tank 31 detected in step S100 and the water temperature set in step S101 to adjust the water temperature of the water 3 stored in the first tank 31 to the designated temperature (step S103).

The scalp cooling apparatus 20 waits until the temperature of the water 3 stored in the first tank 31 reaches the set temperature (No in step S103). When the water temperature of the water 3 stored in the first tank 31 reaches the set temperature (Yes in step S103), the scalp cooling apparatus 20 completes its preparation process (step S104).

When the scalp cooling apparatus 20 completes its preparation process, the backrest 12a in the bed body 12 included in the treatment bed 10 is reclined, whereas the footrest 12b is raised to allow the bed body 12 to move along the rails 13.

The backrest 12a in the treatment bed 10 is reclined to allow the patient 2, who is seated on the treatment bed 10, to lie horizontally on the treatment bed 10, and allows his or her neck 2b to fit on the neck fitting portion 41d included in the base portion 41a.

The cover 41b is then combined with the base portion 41a to cause the sealing sheet 41g to closely fit on the outline of the patient 2 (refer to Fig. 4).

As described above, the preparation process completes when the scalp cooling apparatus 20, which is ready for use, is positioned to surround the scalp 2a of the patient 2. This process can be followed by a treatment process. The cooling treatment that can follow the preparation process will now be described with reference to Fig. 7.

The cooling treatment starts when the water-releasing start controlling unit 64 is operated.

The scalp cooling apparatus 20, which has started the cooling treatment, starts moving the movable water releasing unit 43 (step S110), actuates the first pump 32 (step S111), and also actuates the second pump 52 (step S112).

In detail, the scalp cooling apparatus 20 moves the movable water releasing unit 43 based on the water-releasing mode received by the water-releasing mode controlling unit 67. The actuated first pump 32 starts circulating the water 3 stored in the first tank 31 toward the cooling unit 40, and releases the water 3 that has been adjusted to the set temperature from the water outlets 42 onto the scalp 2a. The water 3 is released in accordance with the releasing amount received by the water-releasing volume controlling unit 66.

The released water 3 is then guided by the second pipe 53a, which is connected to the cooling unit 40, and is collected into the second tank 51.

The water 3 collected into the second tank 51 is then fed back to the first tank 31 by the actuated second pump 52 through the third pipe 53b. This completes one circulation cycle of the water 3 in the scalp cooling apparatus 20.

The scalp cooling apparatus 20 repeats the above circulation cycle of water 3 until the duration time reaches the time received by the water-releasing duration controlling unit 68 (No in step S113).

When the received water-releasing duration time has passed (Yes in step S113), the first pump 32 stops operating (step S114) to stop circulation of the water 3 to the cooling unit 40. Subsequently, the second pump 52 stops operating (step S115) to stop circulation of the water 3 to the first tank 31.

When the first pump 32 and the second pump 52 are stopped, the water temperature adjustment unit 34a stops operating (step S116) to stop the temperature adjustment of the water 3 stored in the first tank 31, and to stop the movable water releasing unit 43 (step S117). This completes the cooling treatment.

Subsequently, the advantages of the scalp cooling method implemented by the scalp cooling system 1 according to the present embodiment will be described.

The scalp cooling system 1 according to the present embodiment includes the treatment bed 10, on which the patient 2 can lie horizontally, and the scalp cooling apparatus 20 described above. The patient 2 can receive treatment without being forced to be in an uncomfortable posture during the cooling treatment.

In the scalp cooling system 1, the bed body 12 is movable to allow the patient 2 to lie on the treatment bed 10 with his or her head being positioned adjacent to the scalp cooling apparatus 20 when the backrest 12a in the bed body 12 of the treatment bed 10 is reclined. This allows the patient 2 to position his or her neck 2b fitting on the neck fitting portion 41d of the base portion 41a simply by reclining the backrest 12a in the bed body 12 of the treatment bed 10, irrespective of the height of the patient 2 seated on the treatment bed 10. This may reduce burdens on the body of the patient 2.

The scalp cooling apparatus 20 according to the present embodiment includes the circulation unit 30, which includes the first tank 31, the first pump 32, and the water temperature adjustment device 34, the cooling unit 40, which includes the cooling apparatus body 41 defining the scalp surrounding space 41c, and the water outlet 42, the collection unit 50, which includes the second tank 51 and the second pump 52, and the operation control unit 60, which includes the operational unit 61 and the controller 62. The scalp cooling apparatus 20 can thus efficiently cool the scalp 2a.

In detail, the circulation unit 30 and the cooling unit 40 allow the water 3 stored in the first tank 31 and adjusted to a set temperature to be released onto the scalp 2a of the patient 2.

The water 3 released onto the scalp 2a of the patient 2 comes in direct contact with the scalp 2a of the patient 2 without an air layer being placed between the water 3 and the scalp 2a of the patient 2, and efficiently cools the scalp 2a of the patient 2 to a predetermined temperature without being affected by the size or the shape of the head of the patient 2.

In this manner, the scalp 2a of the patient 2 who receives chemotherapy treatment through the administration of anticancer drugs can be cooled and maintained at a predetermined temperature. This suppresses the cell dividing activity of cells forming the hair roots, and reduces hair loss, which is a side effect of the administration of the anticancer drugs.

Further, the collection unit 50 allows the water 3 to circulate through the scalp cooling apparatus 20. This structure efficiently uses the circulating water 3 adjusted to the set temperature and uses thermal energy of the water 3 to efficiently adjust the temperature of the scalp 2a, without supplying fresh water 3, which can have a temperature largely different from the temperature of the water 3 stored in the second tank 51.

In detail, without the collection unit 50 for collecting the water 3, an amount of water 3 equal to the amount of water 3 released onto the scalp 2a is to be added to the first tank 31 as appropriate. The added water 3 would change the temperature of the water 3 in the first tank 31. Thermal energy is then needed for adjusting the temperature of the water 3 in the first tank 31 to the set temperature.

The scalp cooling apparatus 20 including the collection unit 50 according to the present embodiment collects the released water 3 into the second tank 51 without wasting it, and reuses the water 3. In other words, the water 3 is circulated to reduce the amount of thermal energy to be used for adjusting the temperature of the water 3 to a predetermined temperature. The water 3 can be reused, and thus fresh water 3 is not added.

Thus, the scalp cooling apparatus 20 efficiently cools the scalp 2a by releasing the water 3 directly onto the scalp 2a, and collects and reuses the released water 3 without discharging and wasting the water 3, and efficiently uses the thermal energy.

The scalp cooling apparatus 20 according to the present embodiment has one water outlet 42 in the surface facing the top part of the head of the patient 2 and in each of the surfaces facing the side parts of the head, among the inner surfaces of the base portion 41a defining the scalp surrounding space 41c. The scalp cooling apparatus 20 further has three water outlets in the front portion of the top surface of the movable water releasing unit 43 at predetermined intervals in the longitudinal direction. This allows the water 3 to be released toward the scalp 2a from the surfaces facing the top part of the head, the side parts of the head, and the back part of the head, and enables more efficient cooling of the scalp 2a.

The scalp cooling apparatus 20 according to the present embodiment includes the movable water releasing unit 43, which has the three water outlets 42, and movable along the groove 41f of the baser portion 41a. The movable water releasing unit 43 with the small number of water outlets can release water 3 onto a wider area of the scalp 2a by moving. The scalp cooling apparatus 20 thus more efficiently cools the scalp 2a.

The scalp cooling apparatus 20 according to the present embodiment includes the controller 62, which controls the first pump 32 based on the releasing amount of the water 3 set by the water-releasing volume controlling unit 66. The scalp cooling apparatus 20 performs efficient cooling that is appropriate for the patient 2 or for the scalp 2a.

In detail, the difference between the predetermined temperature and the temperature of the scalp 2a varies depending on the patient 2 or the scalp 2a. When, for example, the temperature difference is large, a large volume of water 3 may be released. Controlling the releasing volume appropriately for the patient 2 or for the scalp 2a thus enables efficient cooling.

In particular, when the treatment target site is the scalp 2a as in the present embodiment, the head top part of the scalp 2a may be more difficult to cool than the side parts, which are easy to cool. In this case, a large amount of water can be released onto the top part of the head by adjusting the amount of water released from the water outlets 42. This easily adjusts the scalp temperature of the top part of the head, in which the scalp 2a is difficult to cool.

As described above, the amount of the water 3 to be released from the water outlets 42 is adjustable. Thus, the amount of the water 3 to be released can be adjusted in accordance with the treatment target site to efficiently cool the scalp 2a.

The scalp cooling apparatus 20 according to the present embodiment includes the controller 62, which controls the first pump 32 based on the water-releasing duration time of the water 3 set by the water-releasing duration controlling unit 68. This structure can cool the scalp 2a for an intended duration time, or a predetermined time including periods of time before and after the administration of anticancer drugs.

The scalp cooling apparatus 20 according to the present embodiment can also adjust the temperature of the scalp 2a by controlling the releasing amount or the temperature of the water 3 in accordance with the duration time set in accordance with the blood level of an anticancer drug after the drug administration. This prevents excessive cooling of the scalp 2a, and reduces burdens on the patient 2.

The scalp cooling apparatus 20 according to the present embodiment uses the water 3, which is inexpensive and has a high specific heat, as a medium for cooling the scalp 2a. The apparatus thus reduces hair loss, which is a side effect of the administration of anticancer drugs, at low cost.

The structure of the present invention corresponds to the structure according to the above embodiment in the manner described below.

The body surface temperature adjustment apparatus of the present invention including a treatment bed corresponds to the scalp cooling system 1 according to the embodiment.

In the same manner, the treatment target site corresponds to the scalp 2a, the fluid corresponds to the water 3, the treatment bed corresponds to the treatment bed 10, the body surface temperature adjustment apparatus corresponds to the scalp cooling apparatus 20, the storage tank corresponds to the first tank 31, the circulation unit corresponds to the first pump 32 and the second pump 52, the temperature adjustment unit, the first temperature adjustment unit, and the second temperature adjustment unit correspond to the water temperature adjustment device 34, the target site enclosure corresponds to the cooling apparatus body 41, the releasing parts correspond to the water outlets 42, and the collection unit corresponds to the collection unit 50.

However, the present invention is not limited to the structure of the above embodiment, and may be modified variously.

Although the structure described above includes the collection unit 50 including the second tank 51, the second pump 52, the second pipe 53a, and the third pipe 53b, the embodiment is not limited to this structure. For example, the water temperature adjustment device 34, which is included in the circulation unit 30, may be included in the collection unit 50.

This structure allows the released water 3 to be collected into the first tank 31 through the collection unit 50 including the second tank 51 and the water temperature adjustment device 34. The temperature of the water 3 in the first tank 31 can thus be constantly maintained at a predetermined temperature.

In detail, when the released water 3 is directly collected into the first tank 31, the water temperature may change while the water 3 is circulating through the scalp cooling apparatus 20 depending on the amount of the released water or other conditions of use. In that case, the temperature of the water 3 in the first tank 31 can deviate largely from a predetermined temperature.

In the structure with the collection unit 50 including the water temperature adjustment device 34, the water 3 in the second tank 51 can be preliminary adjusted to a predetermined temperature. This prevents the temperature of the water 3 in the first tank 31 from largely deviating from the predetermined temperature due to the water 3 fed to the first tank 31 from the second tank 51. In other words, the water 3 in the first tank 31 can be maintained at a constant temperature.

Thus, the water 3 circulating from the first tank 31 to the cooling unit 40 can constantly be at a set temperature to efficiently cool the scalp 2a.

In the scalp cooling apparatus 20, the water 3 circulates from the circulation unit 30, the cooling unit 40, and to the collection unit 50 in this order. However, the circulation unit 30 and the cooling unit 40 may be connected with the third pipe 53b, and the water 3 may circulate from the circulation unit 30 to the cooling unit 40.

This structure can reduce the number of components of the scalp cooling apparatus 20, and downsize the scalp cooling apparatus 20. However, this structure is preferably used in environments that do not cause the temperature of the water 3 to deviate largely from a predetermined temperature.

In addition to the components described above, the scalp cooling apparatus 20 may further include a filter 54 for filtering the water 3 to be collected between the cooling unit 40 and the collection unit 50, or for example in the second pipe 53a, which is upstream from the second tank.

This structure removes impurities contained in the water 3 to be collected, such as hairs, and maintains the insides of the first tank 31 and the second tank 51 to be clean, and repeatedly releases the clean water 3 onto the scalp 2a.

This structure can also prevent the first pump 32 or the second pump 52 from being clogged with long hairs and being broken, or from lowering the circulation efficiency.

In the structure including the second tank 51 with the filter 54, the second tank 51 including the water temperature adjustment device 34 serves as a sub tank for the first tank 31 when the circulation efficiency decreases. This prevents failures such as insufficient release of the water 3 due to lack of the water 3 that has been adjusted to the set temperature.

In addition to the above components, the scalp cooling apparatus 20 may include a blood level measurement device that can measure the blood level of an anticancer drug. The scalp cooling apparatus 20 sets the water-releasing duration time, the temperature, or the releasing amount of the water 3 in accordance with the blood level of the anticancer drug measured by the blood level measurement device.

This structure can further reduce hair loss that can occur as a side effect of an anticancer drug.

In detail, the time taken by the blood level of an anticancer drug to decrease below a predetermined value differs among individuals. Among the patient 2 who have undergone the cooling treatment for the same duration of time, some patient 2 may have a blood level of the anticancer drug higher than a predetermined value after the cooling treatment. Such patient 2 may have hair loss as a side effect of the anticancer drug.

However, the time taken for the cooling treatment may be set in accordance with the blood level of the anticancer drug instead of being set to a predetermined time. Irrespective of differences between individual subjects, the subjects can continuously receive the cooling treatment until the level of the anticancer drug in their blood decreases below a predetermined value. This can further reduce hair loss that can occur as a side effect of an anticancer drug.

In addition to the components described above, the scalp cooling apparatus 20 may include a scalp temperature detecting unit that detects the temperature of the scalp 2a. The scalp cooling apparatus 20 sets the temperature of the water 3 and the amount of the water 3 released from the water outlets 42 based on the scalp temperature detected by the scalp temperature detecting unit.

This structure can reduce the releasing amount of the water 3 or stop releasing the water 3 when the temperature of the scalp 2a under cooling is lower than the set temperature, and the releasing amount can be increased or the water temperature can be lowered when the temperature of the scalp 2a is higher than the set temperature.

This structure reduces the thermal energy consumed by releasing the water 3, and reduces burdens on the scalp 2a of the patient 2.

In addition to the components described above, the treatment bed 10 may include a heater arranged on the backrest 12a or the bed.

This structure prevents the sensible temperature from being lowered by cooling the scalp, and allows the subject to receive the cooling treatment in a comfortable environment.

The cooling treatment may not be performed on the scalp 2a, and may be performed on body parts of the patient 2 with active cell division, such as finger ends, toes, and areas around eyelids.

The cooling treatment may not be performed using the water 3. The cooling treatment may be performed using a liquid other than the water 3, a gas, a gel, and further a mist of fine droplets of a liquid dispersed in the air, a particulate powder of a solid such as ice, and their mixture.

### REFERENCE SIGNS LIST

- 1: scalp cooling system
- 2: patient
- 2a: scalp
- 2b: neck
- 3: water
- 10: treatment bed
- 11: base
- 12: bed body
- 12a: backrest
- 12b: footrest
- 13: rail
- 20: scalp cooling apparatus
- 30: circulation unit
- 31: first tank
- 32: first pump
- 33: first pipe
- 34: water temperature adjustment device
- 34a: water temperature adjustment unit
- 34b: water temperature detecting unit
- 40: cooling unit
- 41: cooling apparatus body
- 41a: base portion
- 41b: cover
- 41c: scalp surrounding space
- 41d: neck fitting portion
- 41e: outline cut portion
- 41f: groove
- 41g: sealing sheet
- 42: water outlet
- 43: movable water releasing unit
- 50: collection unit
- 51: second tank
- 52: second pump
- 53a: second pipe
- 53b: third pipe
- 60: operation control unit
- 61: operational unit
- 62: controller
- 63: display
- 64: water-releasing start controlling unit
- 65: water temperature controlling unit
- 66: water-releasing amount controlling unit
- 67: water-releasing mode controlling unit
- 68: water-releasing duration controlling unit

## Claims

1. A body surface temperature adjustment apparatus for adjusting a surface temperature of a treatment target site of a patient to a predetermined temperature, the apparatus comprising:
a storage tank configured to store a fluid;
a temperature adjustment unit configured to adjust a temperature of the fluid to the predetermined temperature;
a target site enclosure configured to surround the treatment target site;
a circulation unit configured to circulate the fluid;
a controller configured to control the temperature adjustment unit and the circulation unit;
at least one releasing part configured to release the fluid circulated by the circulation unit toward the treatment target site surrounded by the target site enclosure; and
a collection unit configured to collect the fluid released within a space surrounded by the target site enclosure into the storage tank.

2. The body surface temperature adjustment apparatus according to claim 1, wherein
the at least one releasing part comprises a plurality of releasing parts, and
the plurality of releasing parts are arranged to release the fluid toward the treatment target site in multiple directions.

3. The body surface temperature adjustment apparatus according to claim 1 or claim 2, wherein the at least one releasing part is moveable relative to the treatment target site.

4. The body surface temperature adjustment apparatus according to any one of claims 1 to 3, further comprising:
a fluid-releasing amount controlling unit configured to set a releasing amount of the fluid,
wherein the controller is driven in accordance with the releasing amount set by the fluid-releasing amount controlling unit.

5. The body surface temperature adjustment apparatus according to any one of claims 1 to 4, further comprising:
a fluid-releasing duration controlling unit configured to set a fluid-releasing duration time of the fluid,
wherein the controller is driven in accordance with the fluid-releasing duration time set by the fluid-releasing duration controlling unit.

6. The body surface temperature adjustment apparatus according to any one of claims 1 to 5, wherein the temperature adjustment unit is a first temperature adjustment unit, and
the collection unit includes
a collection tank configured to collect the fluid; and
a second temperature adjustment unit configured to adjust a temperature of the fluid in the collection tank.

7. The body surface temperature adjustment apparatus according to any one of claims 1 to 6, wherein at least one of the target site enclosure or the collection unit includes a filter configured to filter the fluid.

8. The body surface temperature adjustment apparatus according to any one of claims 1 to 7, wherein
the predetermined temperature is lower than a body temperature of the treatment target site,
the fluid is water, and
the treatment target site is a scalp.

9. A body surface temperature adjustment apparatus including a treatment bed, the apparatus comprising:
the body surface temperature adjustment apparatus according to any one of claims 1 to 8; and
a treatment bed configured to allow the patient to lie on horizontally,
wherein the body surface temperature adjustment apparatus is arranged to be adjacent to a head of the patient lying horizontally on the treatment bed.
